(19)

Europäisches
Patentamt
European
Patent Office
Office européen
des brevets

(11) **EP 4 783 172 A1**

(12) **EUROPEAN PATENT APPLICATION**

(43) Date of publication:
**29.07.2026 Bulletin 2026/31**

(21) Application number: **25218207.6**

(22) Date of filing: **25.11.2025**

(51) International Patent Classification (IPC):
**G16B 15/30** $^{(2019.01)}$ **G16B 40/20** $^{(2019.01)}$
**C12Q 1/48** $^{(2006.01)}$

(52) Cooperative Patent Classification (CPC):
**G16B 40/20; G16B 15/30**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **23.01.2025 KR 20250010369
09.05.2025 KR 20250060303**

(71) Applicants:
• **Gachon University of Industry-Academic
Cooperation Foundation
Seongnam-si, Gyeonggi-do 13120 (KR)**

• **Gil Medical Center
Incheon 21565 (KR)**

(72) Inventors:
• **NAM, Seung Yoon
13120 GYEONGGI-DO (KR)**
• **LEE, Jinhyuk
34141 DAEJEON (KR)**
• **LEE, Yeeun
13120 GYEONGGI-DO (KR)**

(74) Representative: **EP&C
P.O. Box 3241
2280 GE Rijswijk (NL)**

(54) **METHOD AND SYSTEM FOR QUANTITATIVE PREDICTION OF BINDING AFFINITY BETWEEN
KINASES AND SMALL MOLECULE COMPOUNDS USING DEEP LEARNING MODELS**

(57)    Proposed are a method of and a system for quantitatively predicting kinase-compound binding affinity using a deep learning (DL) model. This method can accurately and quantitatively predict kinase-compound binding affinity while improving the generalization ability of the model.

EP 4 783 172 A1

# EP 4 783 172 A1

**Description**

CROSS REFERENCE TO RELATED APPLICATION

The present application claims priority to Korean Patent Application No. 10-2025-0010369, filed January 23, 2025 and No. 10-2025-0060303, filed May 09, 2025, the entire contents of which is incorporated herein for all purposes by this reference.

BACKGROUND

1. Technical Field

The present disclosure relates to a method of and a system for quantitatively predicting binding affinity between a kinase and a small-molecule compound using a deep learning (DL) model.

2. Description of the Related Art

Kinases constitute the largest family of enzymes in the human genome and play significant roles in regulating cellular functions through phosphorylation, which is deeply associated with human metabolism. Kinases play crucial roles in various biological pathways, and abnormal activity of kinases can cause numerous diseases. Recent studies on cell signaling have revealed that kinases perform crucial roles during the processes of tumor formation and metastasis. Kinase dysfunction is associated with various malignancies resulting from genetic mutations and chromosomal rearrangements. Therefore, designing compounds that can effectively inhibit kinases may function as a cure for various diseases caused by abnormal activity of kinases. In conclusion, to effectively treat different types of kinase-induced diseases, designing compounds that can inhibit kinases is crucial. Accordingly, studies on drugs targeting kinases have surged, and more than 70 new kinase-targeting drugs were approved by 2021.

As a method of analyzing the activity of kinases, a traditional enzymatic assay involves measuring the enzymatic activity of kinases by incubating purified active kinases with substrates and adenosine triphosphate (ATP) and detecting the degree of phosphorylation. A functional kinase assay is a method of screening kinase inhibitors. However, such kinase activity assays are costly, time-consuming, and face technical challenges in being used in a high-throughput format, which poses a problem.

High-throughput screening (HTS) is technically convenient, provides rapid screening, and is economically advantageous. Hence, inhibitor-binding-based assays using HTS are emerging as alternatives to traditional enzymatic assays. Both enzymatic assays and HTS provide information on the enzymatic activity of kinases and interactions with inhibitors, and are used in a complementary manner.

In the early stages of drug discovery, competition-based HTS was used for broad target profiling, followed by enzymatic assays to more accurately evaluate the functional effects of drugs. Competition-based HTS is a method of quantitatively measuring the binding affinity of small molecules to a large panel of kinases (often several hundreds of different kinases). Competition-based HTS evaluates how well a molecule can bind to different kinases without directly measuring the enzymatic activity of such kinases. This method typically uses a competitive binding assay with an immobilized kinase and a known ligand. Competition-based HTS provides a "binding profile" of a small molecule across a broad spectrum of kinases, which can be advantageously used in the selection of kinase inhibitors by providing information on which kinases a small molecule binds to and to what extent.

In the meantime, artificial intelligence (AI), a technology that enables machines to learn and solve problems like humans, includes subfields such as deep learning (DL) and machine learning (ML).

ML, one field of AI, is a technology that enables machines to learn from data and make predictions. ML uses algorithms that identify patterns from given data and improve through learning. The key elements of ML include: features, which are data attributes used for models to learn; models, which are algorithms that learn from given data and make predictions; and learning, which is the process by which models recognize patterns from data and improve prediction accuracy.

DL, a subfield of ML, solves complex problems through multilayer neural networks based on artificial neural networks (ANN). With large-scale and complex data, DL demonstrates good performance. The key features of DL include multilayer neural networks and self-learning. A multilayer neural network refers to a neural network composed of an input layer, hidden layers, and an output layer for data processing. Additionally, this multilayer neural network extracts features from each layer and learns complex patterns. Furthermore, self-learning refers to the automatic learning of a DL model from large-scale data with numerous parameters.

A convolutional neural network (CNN) model, widely used in DL, is designed to efficiently process two-dimensional (2D) data, such as images, using convolution operations. In a CNN model, the convolutional layer, which is the core part of this model, extracts local features using small kernels. Each kernel generates a feature map at each position, where the kernel weights are trained. The pooling layer performs operations for dimensionality reduction and feature extraction, and max pooling or average pooling is used to reduce the input size of data and computational cost while retaining important information. Next, the fully connected (FC) layer flattens the results obtained through the convolutional and pooling layers into a one-dimensional vector in the final stage of the CNN model for final classification.

At this stage, a probability for each class can be output using functions such as the softmax function, and a continuous value can alternatively be predicted for regression problems. Typically, the rectified linear unit (ReLU) activation function is used in CNN models to introduce nonlinearity, thereby enabling the model to learn more complex patterns.

While binding affinity (for example, percentage kinase inhibition) in competition-based HTS is represented as a continuous value, previous studies on DL have failed to use regression models and directly predict such continuous values. Existing AI methods have focused on predicting binary outcomes, such as binding or non-binding, which are often based on protein chemical X-ray crystallography structures stored in the Protein Data Bank (PDB). Such binary classification models cannot provide information on the strength of binding affinity. Hence, in an era where large-scale HTS data are abundant, there is a need for DL models that can predict kinase-inhibitor binding affinity. Quantitatively predicting interactions between kinases and small-molecule compounds by developing such models is challenging due to the nonlinearity in continuous values of kinase activity inhibition and the high-dimensional nature of the data, and thus remains an ongoing research project.

Recently, DL-based methods have been proposed for predicting protein-ligand binding affinity. For example, in the document [Huiwen Wang. PREDICTION OF PROTEIN-LIGAND BINDING AFFINITY VIA DEEP LEARNING MODELS. Briefings in Bioinformatics. 2024, 25 (2), bbae081], representative types of databases for predicting protein-ligand binding affinity are proposed, together with the information available therefrom, including three-dimensional (3D) structures of protein pocket-ligand complexes, protein kinase sequences, ligand Simplified Molecular Input Line Entry System (SMILES), and ligand secondary structures. In this document, a method of predicting protein-ligand binding affinity through a DL model using neural networks, including a deep fully connected network, a deep convolutional neural network, and a deep recurrent network, is also proposed.

Additionally, in the document [Wern Juin Gabriel Ong. Palani Kirubakaran. John Karanicola. POOR GENERALIZATION BY CURRENT DEEP LEARNING MODELS FOR PREDICTING BINDING AFFINITIES OF KINASE INHIBITORS. bioRxiv. 2023], a model that quantitatively predicts an inhibitory effect on kinases using protein kinase sequences and SMILES of inhibitors through a CNN-based DL model is proposed.

Furthermore, in Korean Patent No. 10-2576033, a method of and a system for predicting protein-ligand binding affinity using a 3D CNN model are proposed.

However, these documents have a problem in that, during the training process of the DL model, the generalization ability of the model is reduced due to excessive optimization of the extreme values. To solve this problem, the present disclosure has improved the generalization ability of a DL model by applying random oversampling (ROS) to values between the extreme values of a training set.

In the meantime, HTS, as a method of analyzing the activity of kinases, is scalable and cost-efficient compared to traditional enzymatic assays. However, most AI models can only determine whether a compound molecule binds to a kinase through binary prediction, and do not measure the binding strength.

Therefore, it is necessary to provide a method that can predict, using an AI model, not only whether a compound molecule binds to a kinase but also how strongly the compound molecule binds to the kinase, without reducing the generalization ability of the model.

In the present disclosure, a method that can accurately and quantitatively predict binding affinity between kinases and inhibitor compounds using DL with HTS data has been developed to enable more accurate and quantitative prediction than binary classification.

SUMMARY

The present disclosure aims to provide a method of and a system for predicting kinase-inhibitor binding affinity, the method and system capable of predicting, using an artificial intelligence (AI) model, not only whether a compound molecule binds to a kinase but also how strongly the compound molecule binds to the kinase, without reducing the generalization ability of the model.

To achieve the above objectives, the present disclosure provides a method of predicting kinase-compound binding affinity, the method being characterized by including the following steps: preparing data on features of a protein kinase, features of a small-molecule compound, and percentage inhibition for a kinase-compound pair; training a deep learning (DL) model with the features of the protein kinase and the features of the compound as input values and with binding affinity of the corresponding kinase-compound pair as an output value; applying random oversampling (ROS) to values between extreme values (ranging from 1% to 99%) of a dataset used in the training step, thereby preserving extreme values of a training set; and predicting and outputting new kinase-compound binding affinity using the trained DL model.

The present disclosure provides a system for predicting kinase-compound binding affinity using a DL model, the system including: a control unit configured to control a DL model; a communication unit capable of communicating with an external server; a memory unit configured to store data; an input unit configured to receive user input; a display unit; and an input/output interface unit configured to control the input unit and the display unit, wherein the memory unit includes data on features of a protein kinase, features of a small-molecule compound, and percentage inhibition for a kinase-compound pair, and the control unit is configured to perform the following steps: training the DL model with the features of the protein kinase and the features of the compound as input values and with binding affinity of the corresponding kinase-compound pair as an output value; and applying ROS to values between extreme values (ranging from 1% to 99%) of a dataset, thereby preserving extreme values of a training set.

The present disclosure can quantitatively and accurately predict kinase-compound binding affinity using high-throughput screening (HTS) data and apply ROS to values between the extreme values during the training process of a DL model to preserve the extreme values of a training set while solving the problem of generalization ability reduced by excessive optimization of the extreme values and improving the generalization ability of the model.

BRIEF DESCRIPTION OF THE DRAWINGS

Figure 1A is a schematic diagram illustrating a high-throughput screening (HTS) assay process for quantitatively measuring binding affinity of a kinase-compound pair;
Figure 1B is a schematic diagram illustrating a process of inhibiting kinase-ligand interactions depending on the degree of binding between kinases and compounds;
Figure 1C is a schematic diagram illustrating data on kinase features used during a training process of a convolutional neural network (CNN) model;
Figure 1D is a schematic diagram illustrating data on compound features used during a training process of a CNN model;
Figure 2A is an overall schematic diagram illustrating a prediction process of kinase-compound binding affinity;
Figure 2B is a diagram illustrating a network architecture of DeepKinome, a deep learning (DL) model used during a prediction process of kinase-compound binding affinity;
Figure 3 is a diagram showing that data on percentage inhibition for kinase-compound pairs is divided and used for training, validation, and testing;
Figure 4 is a diagram showing a comparison of prediction accuracy between DeepKinome used during a prediction process of kinase-compound binding affinity, and other DL and machine learning (ML) models;
Figure 5A is a diagram showing a comparison of root mean squared error (RMSE) values between DeepKinome and XGBoost according to the number of selected features;
Figure 5B is a diagram showing a comparison of Pearson correlation coefficient (PCC) values between DeepKinome and XGBoost according to the number of selected features;
Figure 6A is a diagram showing kinases with the highest PCC values measured by DeepKinome;
Figure 6B is a diagram showing predicted values and actual values of kinase-compound binding affinity for the top five kinases with the highest PCC values measured by DeepKinome;
Figure 6C is a diagram showing the top 10 features that have the greatest influence on the results in a method of predicting kinase-compound binding affinity using DeepKinome;
Figure 7A is a diagram showing chemical diversity and Figure 7B is a diagram showing distribution of kinases and compounds; and
Figure 8 is a schematic diagram illustrating a system for predicting kinase-compound binding affinity.

DETAILED DESCRIPTION

Hereinafter, the present disclosure will be described in detail.
Hereinafter, the present disclosure and embodiments of the present disclosure will be described with reference to the accompanying drawings.
The present disclosure provides a method of predicting kinase-compound binding affinity using a deep learning (DL) model (DeepKinome) that directly regresses the binding affinity of a kinase-compound pair on the basis of features of a protein kinase and features of a compound. The DL model used herein is referred to as DeepKinome, which is a regression model that predicts a continuous output value for new input values by identifying the relationship between the input and output values.

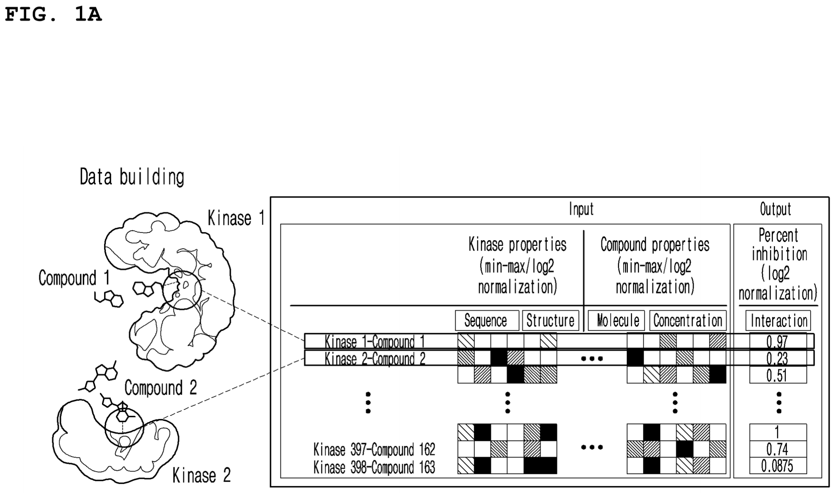

Figure 1A schematically illustrates data for DeepKinome used in the method of predicting kinase-compound binding affinity, according to one embodiment of the present disclosure. In Figure 1A, the data used for training DeepKinome is shown.

**Preparation of Data**

As can be confirmed in the document [Duan Q, Flynn C, Niepel M, Hafner M, Muhlich JL, Fernandez NF, Rouillard AD, Tan CM, Chen EY, Golub TR et al. LINCS CANVAS BROWSER: INTERACTIVE WEB APP TO QUERY, BROWSE AND INTERROGATE LINCS L1000 GENE EXPRESSION SIGNATURES. Nucleic Acids Res 2014. 42(Web Server issue):W449-460], the L1000 database of the "Library of Integrated Network-Based Cellular Signatures (LINCS)" project provides gene expression profile data (that is, KINOMEscan® high-throughput screening (HTS) assay data). From this database, data on 18,771 percentage inhibition values for kinase-compound pairs, including 398 protein kinases and 163 kinase inhibitors (small-molecule compounds), are obtained.

As mentioned in the document [Fabian MA, Biggs WH, 3rd, Treiber DK, Atteridge CE, Azimioara MD, Benedetti MG, Carter TA, Ciceri P, Edeen PT, Floyd M et al. A SMALL MOLECULE-KINASE INTERACTION MAP FOR CLINICAL KINASE INHIBITORS. Nat Biotechnol 2005. 23(3) :329-336], the KINOMEscan® assay, an HTS technique used to study and evaluate the activation and inhibition of kinases, is characterized by being capable of rapidly screening target kinases from large-scale compound libraries. The data on percentage inhibition for the kinase-compound pairs can be obtained through such KINOMEscan® assays. Additionally, data in Simplified Molecular Input Line Entry System (SMILES) format is obtained for the 163 kinase inhibitors. Data in SMILES format is one way of representing chemical structures in string format, and is used to retrieve or store the structures of chemical substances from and in a database.

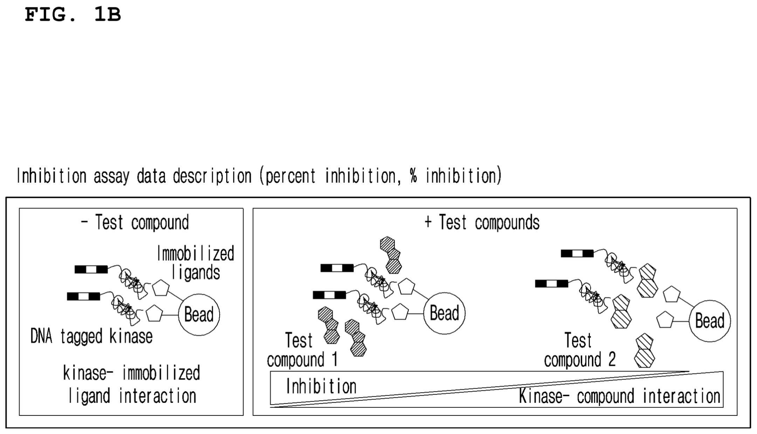

As illustrated in Figure 1B, binding affinity is quantified as percent inhibition (%) of each kinase-compound pair. Percentage inhibition represents the extent to which one compound binds to one kinase active site and can take values ranging from 0% to 100%. While the value of 0% means that the kinase is completely inhibited by the compound, the value of 100% means that the kinase is not inhibited at all by the compound. The terms "binding affinity" and "kinase activity inhibition" herein are used interchangeably. In other words, a high value of kinase-compound binding affinity means that the compound has a significant inhibitory effect on the activity of the kinase. The more strongly the compound binds to the kinase, the greater the inhibitory effect on the kinase because the kinase cannot bind to any ligand. On the contrary, the weaker the extent to which the compound binds to the kinase, the lower the inhibitory effect on the kinase because the kinase can interact with a ligand.

**Input and Output Values of DeepKinome**

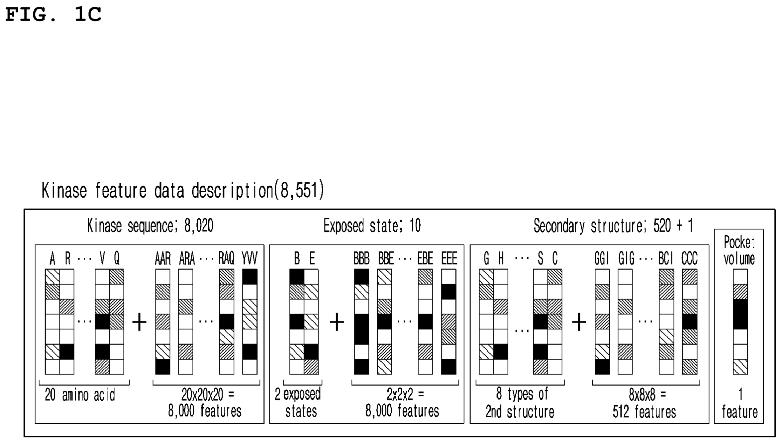

Figure 1C shows 8,551 values of the kinase features used for training DeepKinome. The kinase features in the input vector for the kinase-compound pairs are composed of 8,551 sequence features and molecular features of kinase active sites. As shown in Figure 1C, the kinase features are divided into a total of four categories, each of which represents various protein features calculated for individual kinase residues.

The first category includes 20 individual amino acids (20 features) and 8,000 amino acid 3-mer sequences ($20 \times 20 \times 20$ features) as sequence features of the protein kinases, thereby generating a total of 8,020 features. As mentioned in the document [Kabsch W, Sander C. DICTIONARY OF PROTEIN SECONDARY STRUCTURE: PATTERN RECOGNITION OF HYDROGEN-BONDED AND GEOMETRICAL FEATURES. Biopolymers 1983, 22(12):2577-2637], the "Dictionary of Secondary Structure in Proteins (DSSP)" software, which predicts and analyzes protein secondary structures, is used to identify and classify protein secondary structures on the basis of 3D structures of such proteins. The sequences of the kinase active sites may be obtained from the kinase structures in PDB file format using the "DSSP" software.

The second category includes secondary structure features of the kinase active sites, which may also be obtained using the "DSSP" software. Each amino acid (that is, residue) of the kinase active sites is assigned to one of eight types (G, H, I, T, E, B, S, and C), and the frequencies of these residues are calculated (8 features). Additionally, the frequencies of amino acid 3-mer secondary structure sequences are calculated ($8 \times 8 \times 8 = 512$ features), thereby ultimately obtaining a total of 520 secondary structure features.

The third category includes the frequencies of two features, whether individually exposed or buried, and the surface exposure of the amino acid 3-mers ($2 \times 2 \times 2 = 8$ features) as surface exposure features of protein kinase residues. The surface exposure of each protein kinase residue is defined as follows on the basis of the relative water accessible surface area (RSA) value: RSA = (current water accessible surface area) / (ideal water accessible surface area). Each residue is regarded as exposed when the RSA value is 0.5 or higher, and as buried otherwise.

The last category includes a single feature that represents the volume of kinase-compound interaction sites (pockets).

In summary, a total of 8,551 input features are achieved for the kinases in the kinase-compound pairs.

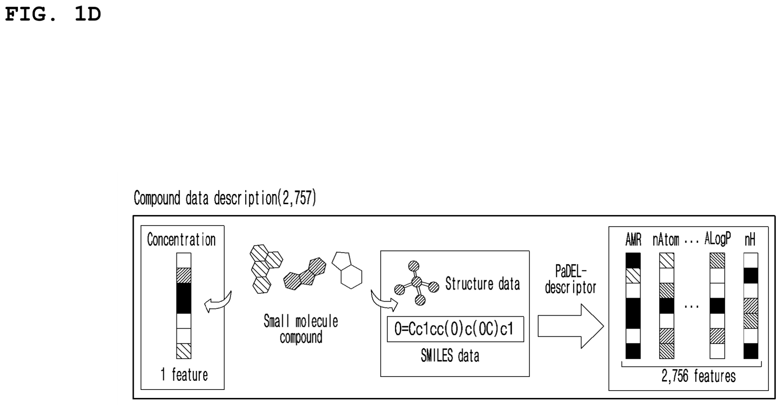

Figure 1D illustrates data on the features of the small-molecule compounds, the data including information on 2,756 chemical molecular structures calculated using the PaDEL molecular fingerprint descriptor software from 2D structure data generated on the basis of the SMILES format of the compounds. The PaDEL molecular fingerprint descriptor software converts the molecular structures of the compounds into molecular fingerprints, such as unique numerical arrays. As a result, 2,756 chemical/molecular structure features of the compounds are derived.

Additionally, information on the concentration of the compounds is included, resulting in a total of 2,757 features in the input vector for the data on the molecular features of the compounds.

As a result, the input vector for the kinase-compound pairs, input into DeepKinome, includes a total of 11,308 features (8,551 kinase features and 2,757 compound features).

When such 11,308 features (8,551 kinase features and 2,757 compound features) are input as input values into DeepKinome in such a manner, kinase-compound binding affinity is calculated as output values.

**Architecture of DeepKinome**

Artificial intelligence (AI) models usable in the present disclosure include DL models and machine learning (ML) models. Preferably, while the DL models include CDRScan-fully connected, CDRScan-master, CDRScan-shallow, CDRScan-tanh, CDRScan-unified, and DeepIC50, the ML models include ridge, lasso, XGBoost, and random forest. More preferably, DeepKinome with a convolutional neural network (CNN) model is used.

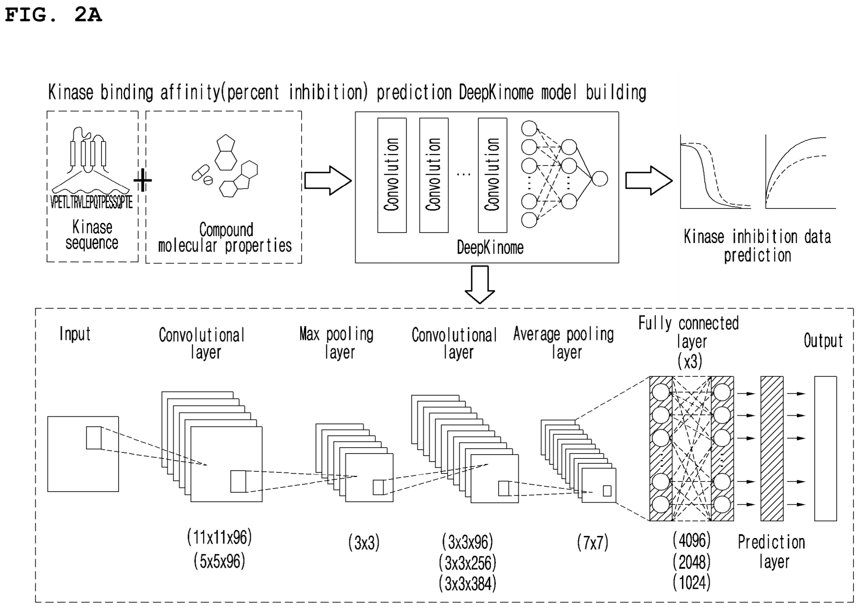

As illustrated in Figure 2A, DeepKinome is a DL-based regression model that predicts kinase activity inhibition by the compounds using a CNN. Table 1 below shows the configuration of DeepKinome for predicting kinase-compound interactions.

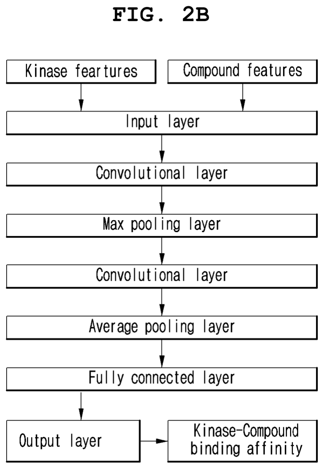

As can be seen in Figure 2B, the CNN model in the present disclosure includes an input layer, convolutional layers, pooling layers, a fully connected layer, and a prediction layer. Specifically, the CNN model includes a convolutional layer, a max pooling layer, a convolutional layer, an average pooling layer, and a fully connected layer. More specifically, the CNN model may include 20 convolutional layers, two pooling layers, and three fully connected layers.

When the input value of the convolutional layer, the output value of each layer, the output position index, and the kernel index are denoted as x, $Conv(x)_{ik}$, i, and k, respectively, a convolution operation may be represented by Equation 1 as

$$Conv(x)_{ik} = F\left(\sum_{m=0}^{m=M-1} w_m^k x_{i+m} + b_{ik}\right)$$

follows: . Here, $b_{ik}$, $w_m^k$, M, and F denote a bias term, the m-th weight of a k-th kernel tensor, the size of the tensor, and an activation function, respectively.

The input value x typically represents a feature map, showing the data input into a network. This value usually corresponds to a 2D or 3D tensor. In $Conv(x)_{ik}$, the output value of each layer, i denotes the output position index, and the i-th position corresponds to a specific position in the result of the convolution operation. Additionally, k denotes the kernel index, and several kernels are used in the convolutional layer to process the input data. Ultimately, $Conv(x)_{ik}$ represents the output value obtained by performing an operation on the input value x using the k-th kernel.

In terms of the bias term $b_{ik}$, one bias is added for each kernel in the CNN model and is summed before applying the activation function, thereby enhancing the learning performance of the model. Additionally, in the kernel weight $w_m^k$, k denotes the kernel index, and m denotes the index of each weight within the kernel. In other words, $w_m^k$ means the m-th weight in the k-th kernel. M denotes the size of the tensor, where the tensor is a multi-dimensional array, and each convolutional layer transforms an input tensor into an output tensor of a specific size.

The activation function F applied to the output value after the convolution operation introduces nonlinearity to the model to enable learning of complex patterns. A representative example thereof is a rectified linear unit (ReLU). Activation functions play important roles in enabling networks to learn nonlinear relationships. The activation function herein may be a ReLU or a linear function. For optimization, the root mean square propagation (RMSprop) optimizer is used. The RMSprop optimizer is an algorithm that performs weight optimization during the training process of the DL model, and serves to efficiently update the parameters of the model by adjusting the learning rate. The learning rate is set to $1 \times 10^{-6}$, and the model is trained for 150 epochs (the number of model training iterations).

The RMSE is used as a loss function, which measures the difference between the predicted value and the actual value obtained from the model, and may serve as an indicator for performance evaluation of the model. The RMSE is a value calculated by squaring the difference between the predicted value and the actual value, calculating the mean, and then taking the square root of the mean value. Accordingly, the lower the RMSE value, the better the predictive performance.

[Table 1]

| Layer | Output Shape | Number of Parameters |
|---|---|---|
| Input layer | (11308 x 1) | 0 |
| Convolutional layer1D_1 | (2827 x 96) | 1152 |
| Batch normalization layer_1 | (2827 x 96) | 384 |
| Activation function_1 | (2827 x 96) | 0 |
| Convolutional layer1D_2 | (1414 x 96) | 46176 |
| Batch normalization layer_2 | (1414 x 96) | 384 |
| Activation function_2 | (1414 x 96) | 0 |
| Max pooling layer | (707 x 96) | 0 |
| Convolutional layer1D_3 | (707 x 96) | 27744 |
| Batch normalization Layer_3 | (707 x 96) | 384 |
| Activation function_3 | (707 x 96) | 0 |
| Convolutional layer1D_4 | (707 x 96) | 27744 |
| Batch normalization layer_4 | (707 x 96) | 384 |
| Activation function_4 | (707 x 96) | 0 |
| Convolutional layer1D_5 | (707 x 256) | 73984 |
| Batch normalization layer_5 | (707 x 256) | 1024 |
| Activation function_5 | (707 x 256) | 0 |
| Convolutional layer1D_6 | (707 x 256) | 196864 |
| Batch normalization layer_6 | (707 x 256) | 1024 |
| Activation function_6 | (707 x 256) | 0 |
| Convolutional layer1D_7 | (707 x 256) | 196864 |
| Batch normalization layer_7 | (707 x 256) | 1024 |
| Activation function_7 | (707 x 256) | 0 |
| Convolutional layer1D_8 | (707 x 256) | 196864 |
| Batch normalization layer_8 | (707 x 256) | 1024 |
| Activation function_8 | (707 x 256) | 0 |
| Convolutional layer1D_9 | (707 x 256) | 196864 |
| Batch normalization layer_9 | (707 x 256) | 1024 |
| Activation function_9 | (707 x 256) | 0 |
| Convolutional layer1D_10 | (707 x 256) | 196864 |
| Batch normalization layer_10 | (707 x 256) | 1024 |
| Activation function_10 | (707 x 256) | 0 |
| Convolutional layer1D_11 | (707 x 384) | 295296 |
| Batch normalization layer_11 | (707 x 384) | 1536 |
| Activation function_11 | (707 x 384) | 0 |
| Convolutional layer1D_12 | (707 x 384) | 442752 |
| Batch normalization layer_12 | (707 x 384) | 1536 |
| Activation function_12 | (707 x 384) | 0 |
| Convolutional layer1D_13 | (707 x 384) | 442752 |

(continued)

| Layer | Output Shape | Number of Parameters |
|---|---|---|
| Batch normalization layer_13 | (707 x 384) | 1536 |
| Activation function_13 | (707 x 384) | 0 |
| Convolutional layer1D_14 | (707 x 384) | 442752 |
| Batch normalization layer_14 | (707 x 384) | 1536 |
| Activation function_14 | (707 x 384) | 0 |
| Convolutional layer1D_15 | (707 x 384) | 442752 |
| Batch normalization layer_15 | (707 x 384) | 1536 |
| Activation function_15 | (707 x 384) | 0 |
| Convolutional layer1D_16 | (707 x 384) | 442752 |
| Batch normalization layer_16 | (707 x 384) | 1536 |
| Activation function_16 | (707 x 384) | 0 |
| Convolutional layer1D_17 | (707 x 256) | 295168 |
| Batch normalization layer_17 | (707 x 256) | 1024 |
| Activation function_17 | (707 x 256) | 0 |
| Convolutional layer1D_18 | (707 x 256) | 196864 |
| Batch normalization layer_18 | (707 x 256) | 1024 |
| Activation function_18 | (707 x 256) | 0 |
| Convolutional layer1D_19 | (707 x 256) | 196864 |
| Batch normalization layer_19 | (707 x 256) | 1024 |
| Activation function_19 | (707 x 256) | 0 |
| Convolutional layer1D_20 | (707 x 256) | 196864 |
| Batch normalization layer_20 | (707 x 256) | 1024 |
| Activation function_20 | (707 x 256) | 0 |
| Average pooling layer | (354 x 256) | 0 |
| Flatten function | (90624) | 0 |
| Fully connected layer_1 | (4096) | 371200000 |
| Activation function | (4096) | 0 |
| Fully connected layer_2 | (2048) | 8390656 |
| Activation function | (2048) | 0 |
| Fully connected layer_3 | (1024) | 2098176 |
| Activation function | (1024) | 0 |
| Prediction layer | (1) | 1025 |

## Training Process of DeepKinome

The architecture of the DL model that predicts kinase-compound binding affinity by preparing data on the kinase features and the compound features and using the prepared data as input values has been described above. Hereinafter, the training process of DeepKinome using this data is to be described in detail.

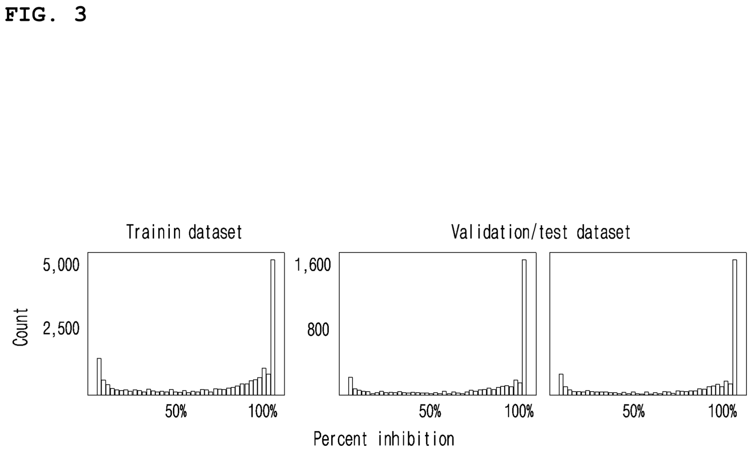

As shown in Figure 3, data on 18,771 percentage inhibition values for the kinase-compound pairs are divided at a ratio of 6:2:2 for training, validation, and testing of DeepKinome, respectively, and used. In other words, the percentage inhibition values are divided into 11,253, 3,759, and 3,759 and used for training, validation, and testing, respectively.

In the training phase, weight optimization is performed to enable the model to learn patterns from the data and make predictions. In this phase, the model is trained with training data. The training data includes input values and the corresponding output values. In the present disclosure, the input values represent values of the kinase features and the compound features, while the output values represent kinase-compound binding affinity. As shown in Figure 3, the data shows a bimodal distribution with peaks at the extremes.

In the validation phase, the model is evaluated to prevent overfitting to the training data, and hyperparameters are adjusted. While training is in progress, the model is periodically evaluated using separate validation data. This validation data is not used for training but is used to evaluate the generalization ability of the model during the training process.

In the testing phase, upon completion of training, the model is evaluated to determine the degree of generalization actually achieved. The model is evaluated in terms of performance using separate test data. This test data is not used at all during the training process of the model and thus plays an important role in measuring actual predictive performance.

As shown in Figure 3, the data shows a bimodal distribution with peaks at the extremes. When the DL model is trained with the extreme values, the model may become overfitted to the extreme values in the training data, and such excessive optimization to the extreme values may reduce the generalization ability of the model. To address this issue, random oversampling (ROS) is applied to values between the extreme values (ranging from 1% to 99%) during the training process, thereby preserving the extreme values of a training set.

ROS is a technique commonly used for imbalanced datasets, in which data of a class (minority class) with a relatively small number of samples is artificially and randomly increased and used for training. As a result, the model is trained more extensively on the minority class, thereby enhancing the predictive performance. In the present disclosure, after conducting the training process using ROS, the total number of training sets is 18,111. The validation set and the test set are left unchanged without applying ROS.

**Performance Comparison with Various AI Models**

To validate the performance of DeepKinome, a comparison with various DL and ML models is conducted. To compare with DeepKinome, the network architectures of the DL models, including CDRScan-fully connected, CDRScan-master, CDRScan-shallow, CDRScan-tanh, CDRScan-unified, and DeepIC50, as well as the ML models, including ridge, lasso, XGBoost, and random forest, are used. CDRScan is a DL network architecture widely used for predicting experimental data on drug screening.

In the case of the DL models, while the input layer is modified to match the input data of the present disclosure, the network architecture is maintained the same as that of the original. For DeepIC50, the activation function for the last layer is modified from the softmax function to a linear activation function. The softmax function takes real numbers as input values and transforms these values into probability values between 0 and 1, enabling probabilistic predictions for each class. Accordingly, the output value is the sum of the probability values for all classes, which is always 1. The linear activation function, mainly used for output layers in regression models, is an activation function used for predicting a continuous value and has no restriction on the output range. To predict the continuous value of kinase-compound binding affinity, the activation function is modified from the softmax function to the linear activation function.

Table 2 shows parameters used to construct the ML models. In the case of random forest among the ML models, grid search optimization is performed to adjust hyperparameters. Similarly, the hyperparameters of the models, lasso, ridge, and XGBoost, are optimized using grid search.

Hyperparameters are external settings for controlling the training of the model. Unlike parameters learned within the model, hyperparameters are set before training and, therefore, affect the performance of the model. To optimize the performance of the model, hyperparameter values are adjusted using grid search, a representative method.

Grid search is a method of exploring all possible combinations of hyperparameters to find the optimal hyperparameter values. First, hyperparameters to be explored are determined, and possible values are set for each hyperparameter, followed by generating all possible combinations of the set hyperparameter values. Then, the model is trained under each combination for performance evaluation.

[Table 2]

| Model | Parameter | Parameter value |
|---|---|---|
| Random forest | Number of estimators | 200 |
| Random forest | Max depth | 30 |
| Random forest | Max feature | sqrt(number of features) |
| Random forest | Min samples leaf | 1 |
| Random forest | Min samples split | 2 |

(continued)

| Random forest | Max feature | sqrt(number of features) |
|---|---|---|
| Ridge | Alpha | 10 |
| Lasso | Alpha | 0.0004 |
| XGBoost | Max depth | 8 |
| XGBoost | Min child weight | 0.001 |
| XGBoost | Gamma | 0.01 |
| XGBoost | Learning rate | 0.1 |

Performance evaluation is conducted on the basis of RMSE and PCC, two indicators calculated for the test dataset. The PCC is an indicator that represents a relationship between the actual value and the predicted value.

The RMSE is a value calculated by squaring the difference between the predicted value and the actual value, calculating the mean, and then taking the square root of the mean value. Accordingly, the lower the RMSE value, the better the predictive performance.

The PCC, a statistical indicator that represents the strength and direction of the linear relationship between two variables, is used to evaluate how strong the relationship between the two variables is, as well as whether the relationship is positive or negative. The higher the PCC value (the closer it is to 1), the stronger the linear relationship between the two variables, meaning that the prediction accuracy in the present disclosure is high.

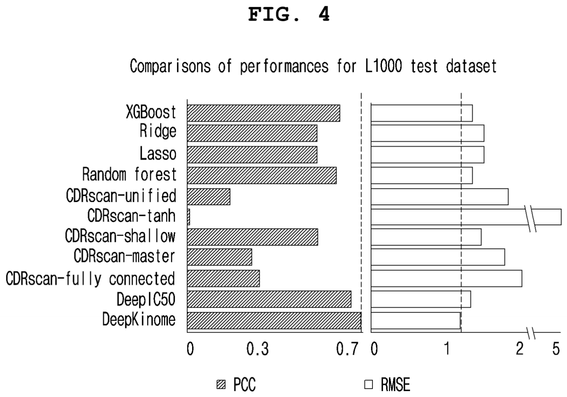

Figure 4 shows a comparison of the predictive performance of DeepKinome and other models. In the L1000 test dataset, the performance of DeepKinome is measured as follows: an RMSE value of 1.156 and a PCC value of 0.732. Overall, the method of predicting kinase-compound binding affinity using DeepKinome demonstrates the best performance among the methods of predicting kinase-compound binding affinity using various DL and ML models. DeepIC50, with the second-best performance, exhibits an RMSE value of 1.278 and a PCC value of 0.692.

The RMSE and PCC values of the ML models (ridge, lasso, random forest, and XGBoost) are in the range of 1.298 to 1.447 and 0.554 to 0.649, respectively.

Among the ML models, random forest and XGBoost demonstrate better performance than lasso and ridge, but are still found to perform worse than DeepKinome.

The RMSE and PCC values of the six DL models are in the range of 1.278 to 5.008 and 0 to 0.697, respectively. Among the DL models, DeepIC50 demonstrates better performance than the other DL models, but still performs worse than DeepKinome.

**Feature Selection**

To determine the optimal number of features for which DeepKinome performs the best when used for predicting kinase-compound binding affinity, DeepKinome and XGBoost (the model evaluated to perform the best among the ML models) are trained with various feature set sizes (2,000, 4,000, 6,000, 8,000, and 10,000). Then, the optimal number of features is identified. The RMSE and the PCC are used as indicators for performance evaluation. Feature selection with random forest is performed using the scikit-learn library in Python. The scikit-learn library offers various feature selection techniques and enables the selection of optimal features suitable for the data using filter methods, wrapper methods, and embedded methods.

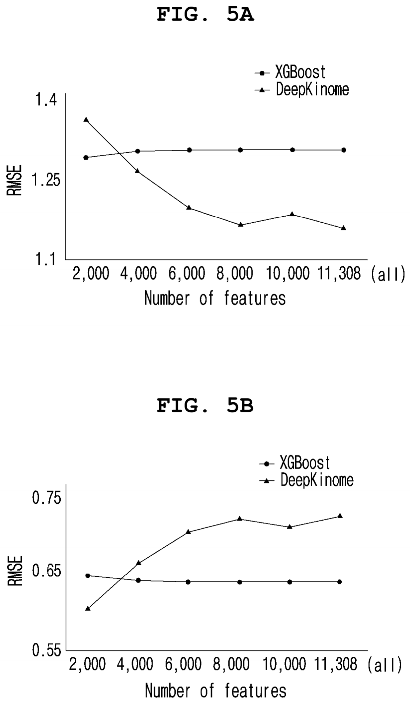

As shown in Figure 5A, it is seen that the RMSE value of XGBoost decreases as the number of features decreases, indicating that the smaller the number of features, the better the performance. On the other hand, it is seen that the RMSE value of DeepKinome decreases as the number of features increases, indicating that the greater the number of features, the better the performance. In other words, this confirms that DeepKinome demonstrates the best performance when using the entire feature set.

As shown in Figure 5B, it is seen that the PCC value of XGBoost increases as the number of features decreases, indicating that the smaller the number of features, the better the performance. On the other hand, it is seen that the PCC value of DeepKinome increases as the number of features increases, indicating that the greater the number of features, the better the performance. In other words, this confirms that DeepKinome demonstrates the best performance when using the entire feature set.

As a result, it is shown that DeepKinome achieves the best performance when using all 11,308 features, whereas XGBoost demonstrates the optimal performance when using 2,000 features, with an RMSE value of 1.286 and a PCC value of 0.653.

The comparative analysis results of DeepKinome and XGBoost in terms of various selected features show that DeepKinome consistently performs better than XGBoost, except at the level of 2,000 features, and exhibits a lower RMSE value and a higher PCC value, resulting in better predictive performance. Furthermore, the greater the number of features, the better the predictive performance.

**Explainable AI (XAI) for Identifying Important Features in Predicting Kinase-Compound Interactions**

In the present disclosure, an attempt is made to understand which features contribute to the accurate prediction of kinase activity inhibition in the kinase-compound pairs. Investigating important features enables the accurate prediction of kinase inhibition, and the method using DeepKinome demonstrates good performance. To evaluate the importance of features, the local interpretable model-agnostic explanation (LIME) method, one of the XAI techniques, is used.

XAI is a technology that enables AI system decision-making processes to be explainable in a human-understandable manner. The LIME method, a technique that explains the prediction of a given instance by training a simple model, not only provides explanations for individual predictions but also offers intuitive reasons why the predictions turn out in that manner.

In the first step, the predicted inhibition values and the actual inhibition values are obtained for all kinase-compound pairs associated with each kinase to calculate the PCC value between the predicted value and the actual value. In this step, the agreement between the predicted percentage inhibition value and the actual value is evaluated.

In the second step, the top five kinases with the highest PCC values are selected. These kinases are considered important features underlying the most accurate model for predicting the percentage inhibition values of the compounds in the dataset.

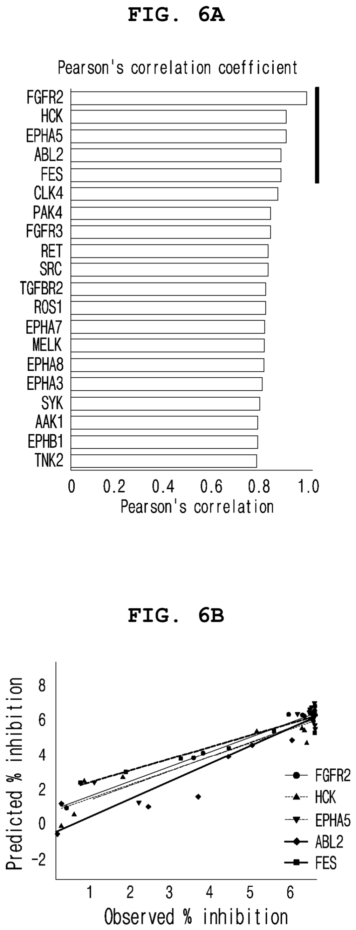

As shown in Figure 6A, the top five kinases with the highest PCC values are selected, and the results thereof are shown as a gray line. The top five kinases are identified as follows: fibroblast growth factor receptor 2 (FGFR2), hematopoietic cell kinase (HCK), EPH receptor A5 (EPHA5), ABL proto-oncogene 2 (ABL2), and FES proto-oncogene, tyrosine kinase (FES).

Figure 6B shows the linear relationship between the predicted inhibition value and the actual inhibition value for the top five kinases with the highest PCC values. This indicates that the predicted inhibition values and the actual inhibition values for the top five kinases with the highest PCC values exhibit a linear relationship on the graph. This shows that the predicted inhibition values for the five kinases are consistent with the actual inhibition values.

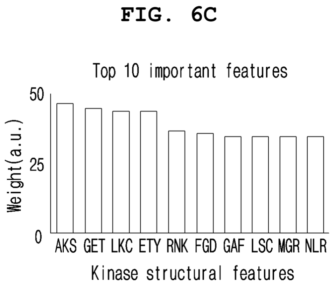

Figure 6C shows the top 10 features that contribute the most to the prediction of kinase-compound percentage inhibition by DeepKinome, as identified through XAI analysis. The top 10 features (AKS, GET, LKC, ETY, RNK, FGD, GAF, LSC, MGR, and NLR) are all amino acid 3-mers. Among these, four features (AKS, GET, ETY, and LSC) contain frequently phosphorylated amino acid residues (S, T, and Y). This indicates that in the method of predicting kinase-compound binding affinity using DeepKinome, phosphorylation-associated amino acid sequences are significantly prioritized when predicting the percentage inhibition of the kinases by the compounds. Furthermore, DeepKinome used in the present disclosure utilizes the amino acid 3-mers, known to be preferred at kinase phosphorylation sites, as important features to predict kinase-compound binding affinity.

**Validation for Chemical Diversity of Dataset**

Predictive performance using AI is essentially related to the diversity of the training data. The present disclosure provides a generalizable prediction method by evaluating the diversity of the compounds and kinases used during the training process of DeepKinome. Accordingly, in the present disclosure, t-distributed stochastic neighbor embedding (t-SNE) is used to evaluate the chemical diversity of the compounds. Furthermore, to evaluate the diversity of the kinases, the distribution of individual kinases is investigated across the overall kinase classification.

t-SNE, a dimensionality reduction technique that transforms high-dimensional data into a low-dimensional representation for visual interpretation, is mainly used to visualize data in 2D or 3D space while preserving the data structure or similarity. While t-SNE maps high-dimensional data into low-dimensional space by modeling the probabilistic similarity between data points, similar data points remain close to each other in the low-dimensional space.

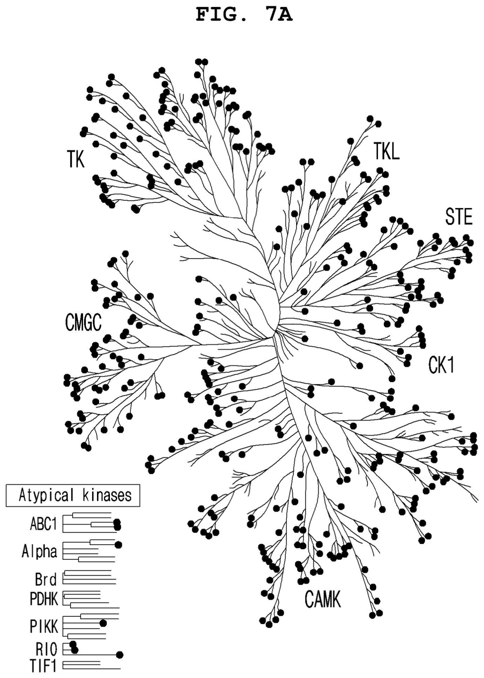

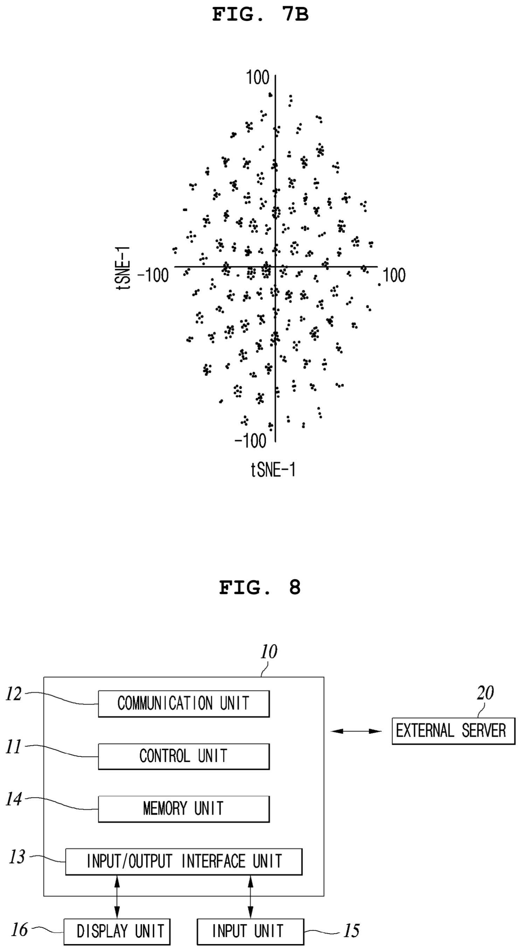

Figure 7A and 7B show that the compounds and kinases used in the present disclosure are not concentrated in any specific part of the kinase classification tree, but are evenly distributed. This indicates that the compounds and kinases used in the present disclosure exhibit high chemical diversity and broad distribution. As a result, in the method of predicting kinase-compound binding affinity using DeepKinome, training is conducted using the kinases and compounds with high chemical diversity, confirming that the predictive performance can be enhanced.

**Kinase-Compound Prediction System**

Figure 8 is a block diagram for describing a computing device and a system for predicting kinase-compound binding affinity according to one embodiment of the present disclosure. Each configuration disclosed in Figure 8 may all be provided in a single device to perform processing independently, or may alternatively be connected via a network such that the respective configurations are executed in separate devices.

An external server 20 may be connected to a prediction system 10 via a network and provide information on sequence features of protein kinases, secondary structure features of kinase active sites, surface exposure features of protein kinase residues, volume features of kinase-compound interaction sites (pockets), molecular structure features of compounds, concentration features of compounds, percentage inhibition for kinase-compound pairs, and the like. For example, the external server 20 may be a database for prediction processing of the prediction system 10 or a server providing the database.

The prediction system 10 may include a control unit 11, a communication unit 12, an input/output interface unit 13, and a memory unit 14.

The control unit 11, configured to control the entire prediction system 10, may, for example, include processing units such as a central processing unit (CPU) and a graphics processing unit (GPU). The control unit 11 can train a DL model using information stored in the memory unit 14 and also calculate a predicted value for new input values through the trained model. Specifically, the control unit 11 can control the model that makes predictions of kinase-compound binding affinity. To this end, the control unit 11 may include a control program such as an operating system (OS), a program defining various processing sequences and the like, and an internal memory for data storage. Furthermore, the control unit 11 may perform information processing to execute various processes by the programs described above.

The communication unit 12 may include an interface connectable to a communication device, such as a router connected to a communication line and the like, and may control communication between the prediction system 10 and the external server 20.

The input/output interface unit 13 may be an interface connected to an input unit 15 and/or a display unit 16. A user can communicate with the prediction system 10 through the input/output interface 13. For example, the display unit 16 may be a means (such as a liquid crystal display, an organic electroluminescence (EL) display, a monitor, or a touch panel) for displaying screens, such as application screens. Additionally, the input unit 15 may, for example, be a key input unit, a touch panel, a control pad (such as a touch pad or a game pad), a mouse, a keyboard, a microphone, or the like.

The memory unit 14 may be a device that stores various databases or tables. For example, the memory unit 14 may include the information on the sequence features of the protein kinases, the secondary structure features of the kinase active sites, the surface exposure features of the protein kinase residues, the volume features of the kinase-compound interaction sites (pockets), the molecular structure features of the compounds, the concentration features of the compounds, the percentage inhibition for the kinase-compound pairs, and the like.

### <Example 1> Method of Predicting Kinase-Compound Binding Affinity Using DL Model

One example of the present disclosure relates to a method of predicting kinase-compound binding affinity using DeepKinome, a DL model.

In one example, the method includes the following steps: preparing data on features of protein kinases, features of small-molecule compounds, and percentage inhibition for kinase-compound pairs; training DeepKinome with the features of the protein kinases and the features of the small-molecule compounds as input values and with binding affinity of the corresponding kinase-compound pairs as output values; applying ROS to values between extreme values (ranging from 1% to 99%) used in the training step, thereby preserving extreme values of a training set; and predicting and outputting new kinase-compound binding affinity.

From the L1000 database (that is, KINOMEscan® HTS assay data) of the "LINCS" project, data on 18,771 percentage inhibition values for the kinase-compound pairs, including 398 protein kinases and 163 kinase inhibitors (small-molecule compounds), were obtained. The data on percentage inhibition for the kinase-compound pairs were obtained through KINOMEscan® assays. Additionally, data in SMILES format was obtained for the 163 kinase inhibitors.

After conducting the training process using ROS, the total number of training sets was 18,111. The validation set and the test set were left unchanged without applying ROS.

### <Example 2> Input and Output Values of DeepKinome

In one example of the present disclosure, the kinase features in the input vector for the kinase-compound pairs may be composed of 8,551 sequence features and molecular features of kinase active sites. The kinase features were divided into a total of four categories, each of which represented various protein features calculated for individual kinase residues.

The first category included 20 individual amino acids (20 features) and 8,000 amino acid 3-mer sequences ($20 \times 20 \times 20$ features) as sequence features of the protein kinases, thereby generating a total of 8,020 features. The sequences of the kinase active sites were obtained from the kinase structures in PDB file format using the "DSSP" software.

The second category included secondary structure features of the kinase active sites, obtained using the "DSSP" software. Each amino acid (that is, residue) of the kinase active sites was assigned to one of eight types (G, H, I, T, E, B, S, and C), and the frequencies of these residues were calculated (8 features). Additionally, the frequencies of amino acid 3-mer secondary structure sequences were calculated ($8 \times 8 \times 8 = 512$ features), thereby ultimately obtaining a total of 520 secondary structure features.

The third category included the frequencies of two features, whether individually exposed or buried, and the surface exposure of the amino acid 3-mers ($2 \times 2 \times 2 = 8$ features) as surface exposure features of protein kinase residues. The surface exposure of each protein kinase residue was defined as follows on the basis of the RSA value: RSA = (current water accessible surface area)/(ideal water accessible surface area). Each residue was regarded as exposed when the RSA value was 0.5 or higher, and as buried otherwise.

The last category included a single feature that represented the volume of kinase-compound interaction sites (pockets).

In summary, a total of 8,551 input features were achieved for the kinases in the kinase-compound pairs.

In one example of the present disclosure, the compound features in the input vector for the kinase-compound pairs may be composed of 2,757 molecular structure features and concentration features.

Data on the features of the small-molecule compounds included information on 2,756 chemical molecular structures calculated using the PaDEL molecular fingerprint descriptor software from 2D structure data generated on the basis of the SMILES format of the compounds. As a result, 2,756 chemical/molecular structure features of the compounds were derived.

Additionally, information on the concentration of the compounds was included, resulting in a total of 2,757 features in the input vector for the data on the molecular features of the compounds.

As a result, the input vector for the kinase-compound pairs, input into DeepKinome, included a total of 11,308 features (8,551 kinase features and 2,757 compound features).

In one example of the present disclosure, when such features are input as input values, kinase-compound binding affinity is calculated as output values.

### <Example 3> Architecture of DeepKinome Using CNN Model

In one example of the present disclosure, DeepKinome may be a DL-based regression model that predicts kinase activity inhibition by the compounds using a CNN.

The CNN model used in one example of the present disclosure may be composed of 20 convolutional layers, two pooling layers, and three fully connected layers. When the input value of each convolutional layer, the output value of each layer, the output position index, and the kernel index are denoted as x, $Conv(x)_{ik}$, i, and k, respectively, a convolution operation may be

$$Conv(x)_{ik} = F\left(\sum_{m=0}^{m=M-1} w_m^k x_{i+m} + b_{ik}\right)$$

represented as follows: . Here, $b_{ik}$, $w^k_m$, M, and F denote a bias term, the m-th weight of a k-th kernel tensor, the size of the tensor, and an activation function, respectively. The activation function may be a ReLU or a linear function. For optimization, the RMSprop optimizer was used, and the RMSE was used as a loss function. The learning rate was set to $1 \times 10^{-6}$, and the model was trained for 150 epochs.

### <Example 4> Performance Comparison of DeepKinome with Various AI Models

In one example of the present disclosure, the method of predicting kinase-compound binding affinity using DeepKinome was compared with other methods of predicting kinase-compound binding affinity using different AI models. To compare with DeepKinome, the network architectures of DL models, including CDRScan-fully connected, CDRScan-master, CDRScan-shallow, CDRScan-tanh, CDRScan-unified, and DeepIC50, as well as ML models, including ridge, lasso, XGBoost, and random forest, were used. CDRScan is a DL network architecture widely used for predicting experimental data on drug screening.

In the case of the DL models, while the input layer was modified to match the input data of the present disclosure, the network architecture was maintained the same as that of the original. For DeepIC50, the activation function for the last layer was modified from the softmax function to a linear activation function.

Performance evaluation was conducted on the basis of two indicators calculated for the test dataset, namely RMSE and PCC. The PCC is an indicator that represents a relationship between the actual value and the predicted value. A lower RMSE value and a higher PCC value indicate better prediction accuracy.

In the L1000 test dataset, the performance of DeepKinome was measured as follows: an RMSE value of 1.156 and a PCC value of 0.732.

The RMSE and PCC values of the ML models (ridge, lasso, random forest, and XGBoost) were in the range of 1.298 to 1.447 and 0.554 to 0.649, respectively.

Among the ML models, random forest and XGBoost demonstrated better performance than lasso and ridge, but were still found to perform worse than DeepKinome.

The RMSE and PCC values of the six DL models were in the range of 1.278 to 5.008 and 0 to 0.697, respectively. Among the DL models, DeepIC50 demonstrated better performance than the other DL models, but still performed worse than DeepKinome.

Overall, the method of predicting kinase-compound binding affinity using DeepKinome demonstrated the best performance among the methods of predicting kinase-compound binding affinity using various DL and ML models.

**<Example 5> Performance Comparison of DeepKinome According to Number of Selected Features**

One example of the present disclosure relates to the method of predicting kinase-compound binding affinity using DeepKinome. To determine the optimal number of features, DeepKinome and XGBoost (the model evaluated to perform the best among the ML models) were trained with various feature set sizes (2,000, 4,000, 6,000, 8,000, and 10,000). Then, performance was evaluated on the test dataset using RMSE. Feature selection with random forest was performed using the scikit-learn library in Python.

It was shown that DeepKinome achieved the best performance when using all 11,308 features, whereas XGBoost demonstrated the optimal performance when using 2,000 features, with an RMSE value of 1.286 and a PCC value of 0.653.

These results confirmed that XGBoost performed better with the smaller number of features, whereas DeepKinome demonstrated the best performance when using the entire feature set.

The comparative analysis results of DeepKinome and XGBoost in terms of various selected features showed that DeepKinome consistently performed better than XGBoost, except at the level of 2,000 features, and exhibited a lower RMSE value and a higher PCC value, resulting in better predictive performance.

**<Example 6> Identification of Important Features in Predicting Kinase-Compound Interactions Using XAI**

In one example of the present disclosure, the importance of features was evaluated to understand which features contributed to the accurate prediction of kinase activity inhibition in the kinase-compound pairs. An XAI technique, the LIME method, was used to evaluate the importance of features used for predicting kinase-compound binding affinity.

In the first step, the predicted inhibition values and the actual inhibition values were obtained for all kinase-compound pairs associated with each kinase to calculate the PCC value between the predicted value and the actual value. In this step, the agreement between the predicted percentage inhibition value and the actual value was evaluated.

In the second step, the top five kinases with the highest PCC values were selected. These kinases were considered important features underlying the most accurate model for predicting the percentage inhibition values of the compounds in the dataset.

As a result of selecting the top five kinases with the highest PCC values, the top five kinases were identified as follows: FGFR2, HCK, EPHA5, ABL2, and FES.

As identified through XAI analysis, the top 10 features that contributed the most to the prediction of kinase-compound percentage inhibition by DeepKinome were AKS, GET, LKC, ETY, RNK, FGD, GAF, LSC, MGR, and NLR, all of which were amino acid 3-mers. Among these, four features (AKS, GET, ETY, and LSC) contained frequently phosphorylated amino acid residues (S, T, and Y). This indicates that in the method of predicting kinase-compound binding affinity using DeepKinome, phosphorylation-associated amino acid sequences are significantly prioritized when predicting the percentage inhibition of the kinases by the compounds. Furthermore, DeepKinome used in the present disclosure utilizes the amino acid 3-mers, known to be preferred at kinase phosphorylation sites, as important features to predict kinase-compound binding affinity.

As a result, the XAI-based feature importance analysis, according to one example of the present disclosure, confirmed that the method of predicting kinase-compound binding affinity using DeepKinome used the phosphorylation-associated amino acid sequences as the key features for deriving the predicted values of kinase-compound binding affinity.

**<Example 7> System for Predicting Kinase-Compound Binding Affinity Using DL Model**

Figure 8 is a block diagram for describing a computing device and a system for predicting kinase-compound binding affinity according to one example of the present disclosure. One example of the present disclosure provides a kinase-compound binding prediction system including a training process of DeepKinome.

In one example, the kinase-compound binding prediction system may be a system for predicting kinase-compound binding affinity using a DL model, the system including: a control unit configured to control a DL model; a communication unit capable of communicating with an external server; a memory unit configured to store data; an input unit configured to receive user input; a display unit; and an input/output interface unit configured to control the input unit and the display unit, wherein the memory unit includes data on features of protein kinases, features of small-molecule compounds, and percentage inhibition for kinase-compound pairs, and the control unit is configured to perform the following steps: training the DL model with the features of the protein kinases and the features of the compounds as input values and with binding affinity of the corresponding kinase-compound pairs as output values; and applying ROS to values between extreme values (ranging from 1% to 99%) of a dataset, thereby preserving extreme values of a training set.

The external server 20 may be connected to the prediction system 10 via a network and provide information on sequence features of the protein kinases, secondary structure features of kinase active sites, surface exposure features of protein kinase residues, volume features of kinase-compound interaction sites (pockets), molecular structure features of the compounds, concentration features of the compounds, percentage inhibition for the kinase-compound pairs, and the like. For example, the external server 20 may be a database for prediction processing of the prediction system 10 or a server providing the database.

The prediction system 10 may include the control unit 11, the communication unit 12, the input/output interface unit 13, and the memory unit 14.

The control unit 11, configured to control the entire prediction system 10, may, for example, include processing units such as a CPU and a GPU. The control unit 11 can train the DL model using information stored in the memory unit 14 and also calculate a predicted value for new input values through the trained model. Specifically, the control unit 11 can control the model that makes predictions of kinase-compound binding affinity. To this end, the control unit 11 may include a control program such as an OS, a program defining various processing sequences and the like, and an internal memory for data storage. Furthermore, the control unit 11 may perform information processing to execute various processes by the programs described above.

The communication unit 12 may include an interface connectable to a communication device, such as a router connected to a communication line and the like, and may control communication between the prediction system 10 and the external server 20.

The input/output interface unit 13 may be an interface connected to the input unit 15 and/or the display unit 16. A user can communicate with the prediction system 10 through the input/output interface 13. For example, the display unit 16 may be a means (such as a liquid crystal display, an organic EL display, a monitor, or a touch panel) for displaying screens, such as application screens. Additionally, the input unit 15 may, for example, be a key input unit, a touch panel, a control pad (such as a touch pad or a game pad), a mouse, a keyboard, a microphone, or the like.

The memory unit 14 may be a device that stores various databases or tables. For example, the memory unit 14 may include the information on the sequence features of the protein kinases, the secondary structure features of the kinase active sites, the surface exposure features of the protein kinase residues, the volume features of the kinase-compound interaction sites (pockets), the molecular structure features of the compounds, the concentration features of the compounds, the percentage inhibition for the kinase-compound pairs, and the like.

**Claims**

1. A computer-implemented method of predicting kinase-compound binding affinity using a deep learning (DL) model, the method comprising:

   preparing data on features of a protein kinase, features of a compound, and percentage inhibition for a kinase-compound pair;
   training a DL model with the features of the protein kinase and the features of the compound as input values and with binding affinity of the corresponding kinase-compound pair as an output value;
   applying random oversampling (ROS) to values between extreme values (ranging from 1% to 99%) of a dataset used in the training of the DL model, thereby preserving extreme values of a training set; and
   predicting and outputting new kinase-compound binding affinity using the trained DL model.

2. The method of claim 1, wherein a convolutional neural network (CNN) model is used as the DL model.

3. The method of claim 1 or 2, wherein the features of the protein kinase comprise a sequence feature of the protein kinase, a secondary structure feature of a kinase active site, a surface exposure feature of a protein kinase residue, and a volume feature of a kinase-compound interaction site (pocket), and
   the features of the compound comprise a molecular structure feature of the compound and a concentration feature of the compound.

4. The method of claim 3, wherein the features of the compound are data generated on the basis of a Simplified Molecular Input Line Entry System (SMILES) format of the compound.

5. The method of claim 1 or 2, wherein a predicted value of the kinase-compound binding affinity output from the DL model is continuous.

6. The method of claim 2, wherein the CNN model comprises 20 convolutional layers, two pooling layers, and three fully connected layers.

7. The method of claim 6, wherein when an input value of the convolutional layer, an output value of each layer, an output position index, a kernel index, a bias term, the m-th weight of a k-th kernel tensor, a size of the tensor, and an activation function are denoted as x, $Conv(x)_{ik}$, i, k, $b_{ik}$, $w^k_m$, M, and F, respectively, a convolution operation is represented as follows:

$$Conv(x)_{ik} = F\left(\sum_{m=0}^{m=M-1} w^k_m x_{i+m} + b_{ik}\right)$$

.

8. The method of claim 7, wherein the activation function is a rectified linear unit (ReLU) or a linear function.

9. The method of claim 1 or 2, wherein as the number of features used in the training of the DL model increases, a root mean squared error (RMSE) value decreases, and a Pearson correlation coefficient (PCC) value increases.

10. The method of claim 2, wherein the training of the CNN model is to train the CNN model using 10,000 or more features.

11. The method of claim 1, wherein, as a result of explainable AI (XAI)-based feature importance analysis, the method uses a phosphorylation-associated amino acid sequence as the most important feature for deriving a predicted value of the kinase-compound binding affinity.

12. The method of claim 11, wherein the phosphorylation-associated amino acid sequence is an amino acid 3-mer.

13. The method of claim 12, wherein the amino acid 3-mer comprises one or more of serine (S), threonine (T), and tyrosine (Y).

14. A system for predicting kinase-compound binding affinity using a DL model, the system comprising:

a control unit configured to control a DL model;
a communication unit capable of communicating with an external server;
a memory unit configured to store data;
an input unit configured to receive user input;
a display unit; and
an input/output interface unit configured to control the input unit and the display unit,
wherein the memory unit comprises data on features of a protein kinase, features of a small-molecule compound, and percentage inhibition for a kinase-compound pair, and
the control unit is configured to perform: training the DL model with the features of the protein kinase and the features of the compound as input values and with binding affinity of the corresponding kinase-compound pair as an output value; and applying ROS to values between extreme values (ranging from 1% to 99%) of a dataset, thereby preserving extreme values of a training set.

15. The system of claim 14, wherein a CNN model is used as the DL model.

FIG. 1A

Data building

EP 4 783 172 A1

**FIG. 1B**

Inhibition assay data description (percent inhibition, % inhibition)

FIG. 1C

Kinase feature data description(8,551)

FIG. 1D

Compound data description(2,757)

Concentration

1 feature

Small molecule compound

Structure data

O=Cc1cc(O)c(OC)c1

SMILES data

PaDEL-descriptor

AMR  nAtom ... ALogP  nH

2,756 features

FIG. 2A

Kinase binding affinity(percent inhibition) prediction DeepKinome model building

Kinase
sequence

VPELTRVLEPGTPESSDPTE

+

Compound
molecular properties

→

DeepKinome

Convolution
Convolution ... Convolution

→

Kinase inhibition data
prediction

Output

Prediction layer
(4096)
(2048)
(1024)

Fully connected
layer
(x3)

(7x7)

Average pooling
layer

(3x3x384)
(3x3x256)
(3x3x96)

Convolutional
layer

(3x3)

Max pooling
layer

(5x5x96)

Convolutional
layer

(11x11x96)

Input

## FIG. 2B

FIG. 3

# FIG. 4

Comparisons of performances for L1000 test dataset

FIG. 5A

FIG. 5B

## FIG. 6A

## FIG. 6B

FIG. 6C

Top 10 important features

## FIG. 7A

## FIG. 7B

## FIG. 8

| | Europäisches Patentamt European Patent Office Office européen des brevets | **EUROPEAN SEARCH REPORT** | **Application Number** EP 25 21 8207 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WANG HUIWEN ET AL: "DLSSAffinity: protein-ligand binding affinity prediction i via /i a deep learning model", PHYSICAL CHEMISTRY CHEMICAL PHYSICS, vol. 24, no. 17, 4 April 2022 (2022-04-04) , pages 10124-10133, XP093392205, ISSN: 1463-9076, DOI: 10.1039/d1cp05558e * pp. 10124, 10125, 10126, 10128, 10131 * ----- | 1-15 | INV. G16B15/30 G16B40/20 ADD. C12Q1/48 |
| A | WANG HUIWEN: "Prediction of protein-ligand binding affinity via deep learning models", BRIEFINGS IN BIOINFORMATICS, vol. 25, no. 2, 22 January 2024 (2024-01-22), XP093392070, GB ISSN: 1467-5463, DOI: 10.1093/bib/bbae081 * whole document, in particular p. 7 * ----- | 1-15 | |
| A | D'SOUZA SOFIA ET AL: "Deep Learning-Based Modeling of Drug-Target Interaction Prediction Incorporating Binding Site Information of Proteins", INTERDISCIPLINARY SCIENCES: COMPUTATIONAL LIFE SCIENCES, vol. 15, no. 2, 26 March 2023 (2023-03-26) , pages 306-315, XP093392206, CA ISSN: 1913-2751, DOI: 10.1007/s12539-023-00557-z * whole document, in particular p.309, section "2.3 Feature Selection of Proteins" * ----- -/-- | 1-15 | **TECHNICAL FIELDS SEARCHED (IPC)** G16B C12Q |

The present search report has been drawn up for all claims

| Place of search | Date of completion of the search | Examiner |
|---|---|---|
| Munich | 28 April 2026 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Europäisches
Patentamt

European
Patent Office

Office européen
des brevets

Application Number

**EP 25 21 8207**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | LEE YEEUN ET AL: "DeepKinome: quantitative prediction of kinase binding affinity by a compound using deep learning based regression model", FRONTIERS IN MOLECULAR BIOSCIENCES, vol. 12, 17 December 2025 (2025-12-17), XP093391984, Switzerland ISSN: 2296-889X, DOI: 10.3389/fmolb.2025.1698891 * the whole document * | 1-15 | |
| A | TRIPATHI MANISH KUMAR ET AL: "Evolving scenario of big data and Artificial Intelligence (AI) in drug discovery", MOLECULAR DIVERSITY SPRINGER INTERNATIONAL PUBLISHING, CHAM, vol. 25, no. 3, 23 June 2021 (2021-06-23), pages 1439-1460, XP037530189, ISSN: 1381-1991, DOI: 10.1007/S11030-021-10256-W [retrieved on 2021-06-23] * whole document, in particular p. 1447 * | 1-15 | |

TECHNICAL FIELDS SEARCHED (IPC)

The present search report has been drawn up for all claims

| Place of search | Date of completion of the search | Examiner |
|---|---|---|
| Munich | 28 April 2026 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020250010369
- KR 1020250060303
- KR 102576033

**Non-patent literature cited in the description**

- **PALANI KIRUBAKARAN** ; **JOHN KARANICOLA**. POOR GENERALIZATION BY CURRENT DEEP LEARNING MODELS FOR PREDICTING BINDING AFFINITIES OF KINASE INHIBITORS. *bioRxiv*, 2023
- **DUAN Q** ; **FLYNN C** ; **NIEPEL M** ; **HAFNER M** ; **MUHLICH JL** ; **FERNANDEZ NF** ; **ROUILLARD AD** ; **TAN CM** ; **CHEN EY** ; **GOLUB TR et al.** LINCS CANVAS BROWSER: INTERACTIVE WEB APP TO QUERY, BROWSE AND INTERROGATE LINCS L1000 GENE EXPRESSION SIGNATURES. *Nucleic Acids Res*, 2014, vol. 42, W449-460
- **FABIAN MA** ; **BIGGS WH, 3RD** ; **TREIBER DK** ; **ATTERIDGE CE** ; **AZIMIOARA MD** ; **BENEDETTI MG** ; **CARTER TA** ; **CICERI P** ; **EDEEN PT** ; **FLOYD M et al.** A SMALL MOLECULE-KINASE INTERACTION MAP FOR CLINICAL KINASE INHIBITORS. *Nat Biotechnol*, 2005, vol. 23 (3), 329-336
- **KABSCH W** ; **SANDER C**. DICTIONARY OF PROTEIN SECONDARY STRUCTURE: PATTERN RECOGNITION OF HYDROGEN-BONDED AND GEOMETRICAL FEATURES. *Biopolymers*, 1983, vol. 22 (12), 2577-2637